# EUROPEAN PATENT SPECIFICATION

(11) **EP 3 479 833 B1**
(45) Date of publication and mention of the grant of the patent: **01.11.2023**
(21) Application number: 17820351.9
(22) Date of filing: 30.06.2017
(51) Int. Cl.: A61K 35/545, A61P 1/00, A61P 1/16, A61P 5/00, A61P 9/00, A61P 11/00, A61P 13/00, A61P 15/00, A61P 17/00, A61P 25/00

(54) **PROPHYLACTIC OR THERAPEUTIC AGENT FOR ORGAN FIBROSIS**
PROPHYLAKTISCHES ODER THERAPEUTISCHES MITTEL GEGEN ORGANFIBROSE
AGENT DE TRAITEMENT PROPHYLACTIQUE OU THÉRAPEUTIQUE POUR LA FIBROSE DES ORGANES

(30) Priority: 01.07.2016 JP 2016132075
(43) Date of publication of application: 08.05.2019
(73) Proprietor: Tohoku University, Sendai-shi, Miyagi 980-8577 (JP)
(72) Inventor: DEZAWA, Mari, Sendai-shi, Miyagi 980-8577 (JP); UNNO, Michiaki, Sendai-shi, Miyagi 980-8577 (JP); YAMAMOTO, Toshihiro, Tokyo 101-8517 (JP); SHINDO, Yasuhiro, Tokyo 101-8517 (JP); HARA, Hiroto, Tokyo 101-8517 (JP); MASUTOMI, Naoya, Tokyo 101-8517 (JP)
(74) Representative: Gill Jennings & Every LLP
(86) International application number: PCT/JP2017/024246
(87) International publication number: WO 2018/003997

(56) References cited:
- WO-A1-2008/140141
- WO-A1-2008/148105
- WO-A1-2014/027684
- WO-A1-2014/089397
- WO-A1-2016/054155
- WO-A2-2014/123930
- SHUICHI KANEKO: "C-gata Kan'en kara Hatsugan ni Itaru Byotai Shinten no Kaimei to sono Seigyo ni Kansuru Kenkyu", Kosei Rodo Kagaku Kenkyuhi Hojokin Kan'en To Kokufuku Jitsuyoka Kenkyu Jigyo, Heisei 26 Nendo Sokatsu · Buntan Kenkyu Hokokusho, 2015, pages 1-11, XP009518761,
- ZHAO, W. et al.: "Intravenous injection of mesenchymal stem cells is effective in treating liver fibrosis", World J. Gastroenterology, vol. 18, no. 10, 2012, pages 1048-1058, XP055452379,
- NORIYUKI KATSUNO et al.: "Treatment of refractory kidney diseases using adipose tissue-derived stromal cells", Journal of Clinical and Experimental Medicine, vol. 242, no. 4, 2012, pages 289-295, XP009514178, ISSN: 0039-2359
- LI, W. et al.: "Isogenic mesenchymal stem cells transplantation improves a rat model of chronic aristolochic acid nephropathy via upregulation of hepatic growth factor and downregulation of transforming growth factor beta1", Mol. Cell . Biochem., vol. 368, no. 1-2, 4 June 2012 (2012-06-04), pages 137-145, XP035091077,
- ISEKI, M. et al.: "Human Muse Cells, Nontumorigenic Pluripotent-Like Stem Cells, Have Liver Regeneration Capacity Through Specific Homing and Cell Replacement in a Mouse Model of Liver Fibrosis", Cell Transplantation, vol. 26, no. 5, 1 May 2017 (2017-05-01), pages 821-840, XP055453778, ISSN: 0963-6897, DOI: 10.3727/096368916X693662
- MARI DEZAWA: "Muse Saibo ga Motarasu Iryo Kakushin", Dai 14 Kai Japan Research Association for Immunotherapeutics Gakujutsu Shukai Program ·Shorokushu; 14th Annual Meeting of Japan Research Association of Immunotherapists; 11/02/2017, 27 January 2017 (2017-01-27), pages 14-15, XP009518615, JAPAN

## Description

### TECHNICAL FIELD

The present invention relates to a cell product for regenerative therapy. More particularly, the present invention relates to a cell product comprising a pluripotent stem cell effective in repairing and regenerating organs where fibrosis has occurred.

### BACKGROUND ART

Organ fibrosis is known to occur due to insufficient regeneration of the organs and fibrosis by increased connective tissues after injury, necrosis, and the like of organs caused by, for example, infection, inflammation, accumulation of endogenous substances such as fat, and denaturation of tissues and cells, by various causes such as microorganisms, chemicals, *in vivo* responses such as immune response, food habits, environment, and inheritance (including an unknown cause). Organ fibrosis are also known to occur in various organs such as liver, lung, heart, kidney, and central nervous system, as well as many organs and tissues such as muscle, bone and skin.

Liver cirrhosis, a fibrosis occurring in liver, is a pathological condition in which liver diseases induced by various causes finally reach at the end of chronic progression, and observed are decrease in the number of functional hepatocytes and increase in fibrous tissue, remarkably impairing liver functions. Despite the reported number of patients with liver cirrhosis being about 300 thousand in Japan and about 20 million in the world, no effective medical treatment for serious hepatic failure due to liver cirrhosis has been established. The current medical therapy is mainly targeted to delay the progression of chronic liver disease to liver cirrhosis by using various symptomatic treatments.

Pulmonary fibrosis is a poor-prognosis disease that is accompanied with cough, chest pain and dyspnea, etc., and includes fibrosis caused by lung injury associated with pneumonia such as interstitial pneumonia, as well as idiopathic pulmonary fibrosis with an unknown cause. Myocardial fibrosis, a fibrosis in myocardium or cardiac valve tissue caused by primary diseases such as cardiomyopathy due to coronary circulation failure and infection/immune reaction, and valvular disease, leads to heart failure when it progresses.

In kidney, fibrosis in kidney tissue caused by progression of chronic kidney disease rapidly worsens renal functions, resulting in an irreversible state. As described above, it has been known that progression of primary diseases in various organs and tissues may cause fibrosis of the organs and tissues. In any of the diseases, the progression of fibrosis causes an extremely poor-prognostic condition of disease that manifests irreversible functional impairments in the organs and tissues and that is difficult to be treated by current medicine.

Liver transplant is the only effective treatment for liver cirrhosis, but has many problems, such as lack of organ donor, high medical cost, and risks to donor in the case of living donor liver transplant. Despite the year-by-year increase in the patients waiting for a liver transplant, increase in the number of death while waiting for transplant due to shortage of organ donor is also a major issue.

In recent years, stem cell transplantation has attracted attention as a therapy which can replace transplantation therapy against diseased organs. For example, mesenchymal stem cells (MSCs) have been reported to suppress liver fibrosis and inflammation in chronic liver disease (e.g., Non-Patent Document 1). Since MSCs are less frequent in engraftment to injured liver tissues and differentiation to new hepatocytes, MSC has fibrogenesis-inhibiting action and suppressive effects on inflammation through the mechanism of anti-inflammatory effect and production of inhibitory factors of fibrosis and protective factors (e.g., Non-Patent Document 2). The studies of transplantation of embryonic stem cells (ES cells) - or induce pluripotent stem cells (iPS cells)-induced hepatic progenitor cells into liver have been developed, however, there remains critical issues to be overcome, such as contamination of undifferentiated cells and neoplastic transformation due to genomic instability. Therefore, a fundamental solution by efficient stem cell therapy to restore functional hepatocytes is desired.

Studies by Dezawa, one of the present inventors, has revealed that pluripotent stem cells (Multilineage-differentiating Stress Enduring cells; Muse cell), which exist in mesenchymal cell fractions and can be obtained without gene transferor induction by cytokines or the like and express SSEA-3 (Stage-Specific Embryonic Antigen-3) as a surface antigen, can be responsible for the pluripotency possessed by the mesenchymal cell fractions, and can be applied to disease treatment aimed at tissue regeneration (Patent Document 1; Non-Patent Documents 3 to 5). However, it has not been demonstrated whether the use of Muse cells in prevention and/or treatment of fibrosis could provide the expected therapeutic effects.

### PRIOR ART REFERENCES

### PATENT DOCUMENT

Patent Document 1: Japanese Patent No. 5185443

### NON-PATENT DOCUMENTS

Non-Patent Document 1: Transplantation 2004; 78: 83-88
Non-Patent Document 2: The Journal of surgical research 2014; 186: 408-416
Non-Patent Document 3: Proc. Natl. Acad. Sci. USA, 2010; 107: 8639-8643
Non-Patent Document 4: Proc. Natl. Acad. Sci. USA, 2011; 108: 9875-9880
Non-Patent Document 5: Nat. Protc., 2013; 8: 1391-1415

### SUMMARY OF THE INVENTION

### PROBLEMS TO BE SOLVED BY THE INVENTION

An object of the present invention is to provide a cell product for prevention and/or treatment of organ fibrosis.

### MEANS FOR SOLVING THE PROBLEMS

The present inventors have found that in a liver fibrosis model using an immunodeficient mouse that does not reject human cells, intravascularly-administrated human Muse cells accumulated and engrafted to the injured liver, restored and repaired the injured liver, and improved or recovered the liver functions, and thus that the Muse cells can be suitably used in treatment and prevention of organ fibrosis including liver fibrosis, thereby completed the present invention.

Accordingly, the present invention provides the following [1] to [10].
[1] A cell product for prevention and/or treatment of organ fibrosis, comprising a SSEA-3-positive pluripotent stem cell derived from a mesenchymal tissue or cultured mesenchymal cell.
[2] The cell product of item [1], wherein said organ fibrosis is a fibrosis occurring in digestive organ, respiratory organ, cardiovascular organ, urogenital organ, locomotor organ, central nervous system, or endocrine organ, or on skin.
[3] The cell product of item [2], wherein said organ fibrosis is a fibrosis occurring in digestive organ.
[4] The cell product of item [3], wherein said organ fibrosis is liver fibrosis.
[5] The cell product of item [4], wherein said pluripotent stem cell is capable of differentiating into a cell expressing a hepatoblast marker or hepatocyte marker.
[6] The cell product of item [4] or [5], which improves serum total bilirubin and/or serum albumin levels when administered to a subject as compared with that of non-administration group.
[7] The cell product of item [2], wherein said organ fibrosis is a fibrosis occurring on skin.
[8] The cell product of item [2], wherein said organ fibrosis is a fibrosis occurring in lung.
[9] The cell product of any one of items [1] to [8], wherein said pluripotent stem cell is one having all of the following characteristics:
   (i) having low or no telomerase activity;
   (ii) capable of differentiating into any of triploblastic cells;
   (iii) showing no neoplastic proliferation; and
   (iv) having self-renewal capacities.
[10] A cell product for inhibition of tissue fibrosis and/or lysis of fibrotic tissue, comprising a SSEA-3-positive pluripotent stem cell derived from a living mesenchymal tissue or a cultured mesenchymal cell.

### EFFECT OF THE INVENTION

In the present invention, Muse cells are administered to a subject with fibrosis via vascular or the like, or directly to the target organ and its surroundings. Then, the Muse cells accumulating in the injured organ with fibrosis suppress the progression of fibrosis or lyse fibrous tissues that have been already established, while spontaneously differentiating into cells constituting the organ. Through such a regeneration mechanism, the Muse cells can eliminate fibrous tissues due to fibrosis and improve organ functions.

Since Muse cells can efficiently migrate and engraft to organs such as injured liver, and then spontaneously differentiate into liver-constituting cells such as hepatocyte in the engraftment site, they do not require differentiation induction into therapeutic target cells prior to transplantation. In addition, Muse cells are non-tumorigenic and superior in safety. Furthermore, since Muse cells are not subjected to immune rejection, treatment with allogenic cell product produced from donors is also possible. Therefore, the Muse cells having the superior abilities as described above can provide easy and feasible means for treatment of patients with organ fibrosis.

### BRIEF DESCRIPTION OF THE DRAWINGS

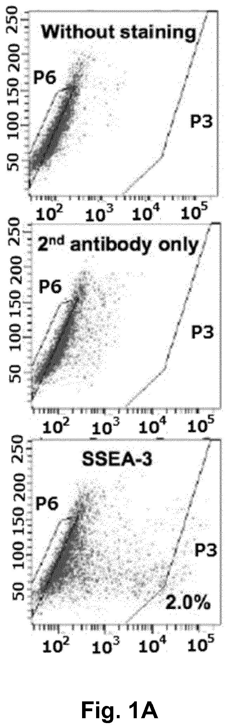

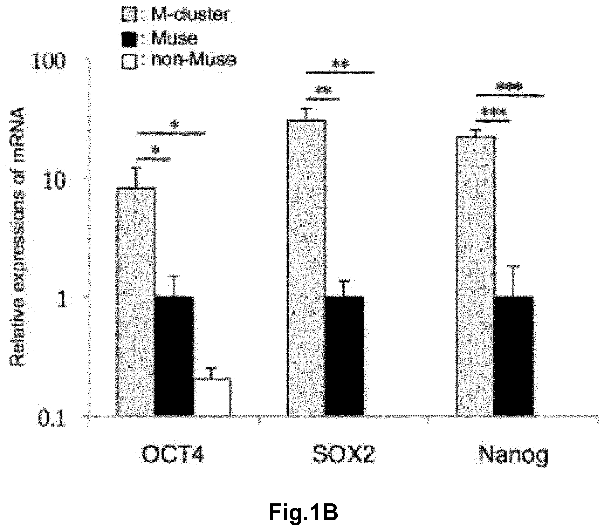

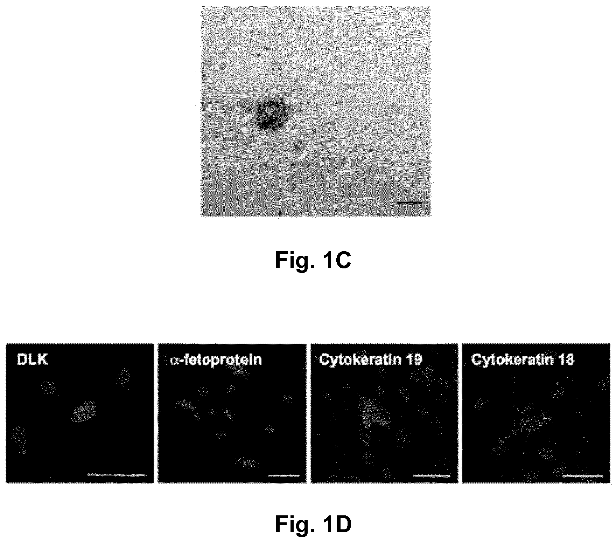

FIG. 1 shows characterization of Muse cell. (A) SSEA-3 (+)-Muse cells (gate P3) and SSEA-3 (-)-non-Muse cells (gate P6) sorted from human BM-MSC. Top panel: without staining; middle panel: secondary antibody only; and bottom panel: anti-SSEA-3 antibody. (B) Results of Q-PCR for OCT4, SOX2, and Nanog in M-cluster, Muse cell, and non-Muse cell. *: P < 0.05, : P < 0.01, : P < 0.001. (C) A photomicrograph of cells grown from a single M-cluster on a gelatin-coated dish. (D) A photomicrograph showing the results of immunostaining of cells grown from a single M-cluster on a gelatin-coated dish. DLK, α-fetoprotein, cytokeratin 19, and cytokeratin 18 were used as markers. Scale bar: 50 µm.
FIG. 2 shows results for in vitro migration of Muse cells to serum and liver obtained from animals before (intact) and 1, 24, 48 hours after administration of carbon tetrachloride (CCl₄). Muse cells migrated to (A) serum and (B) liver tissue with higher efficiency as compared to migration observed in non-Muse cells. ***p < 0.001.

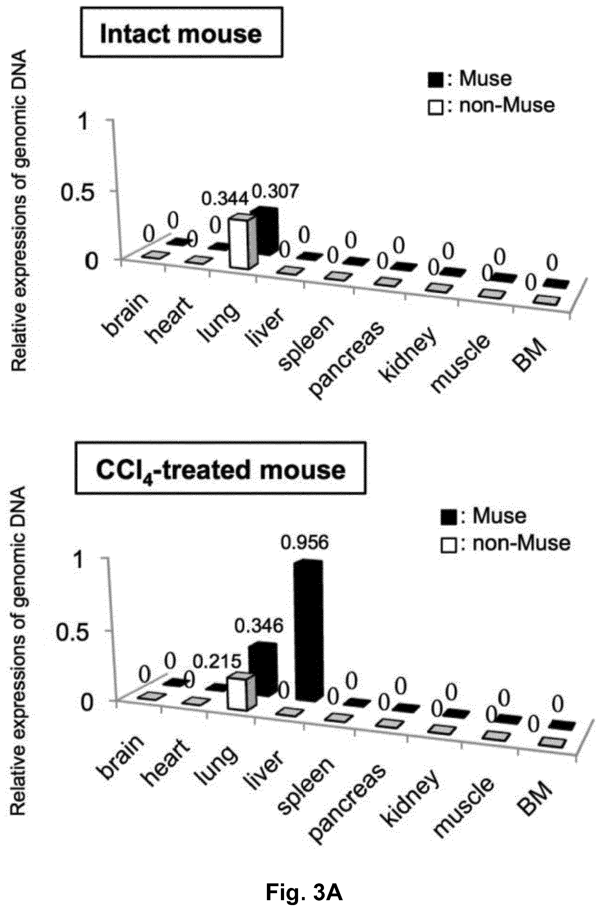

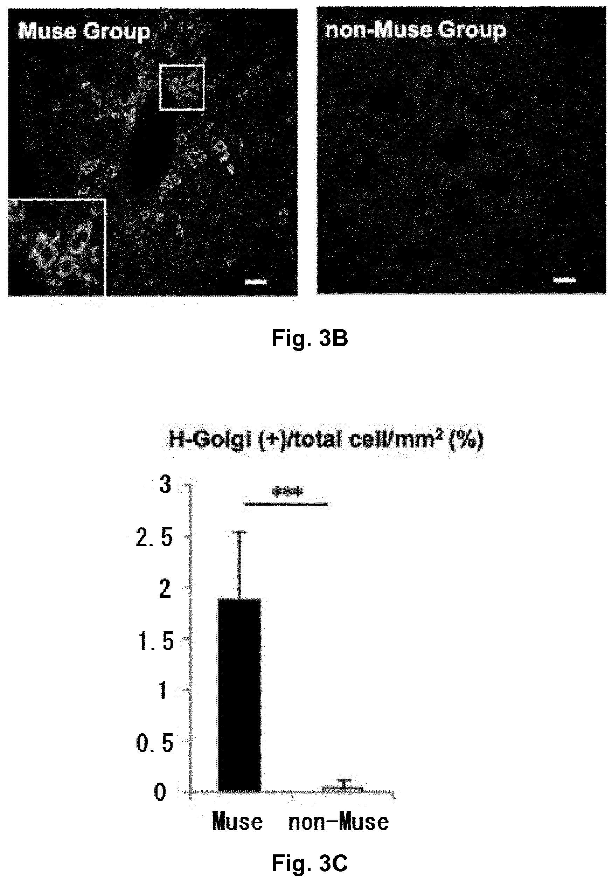

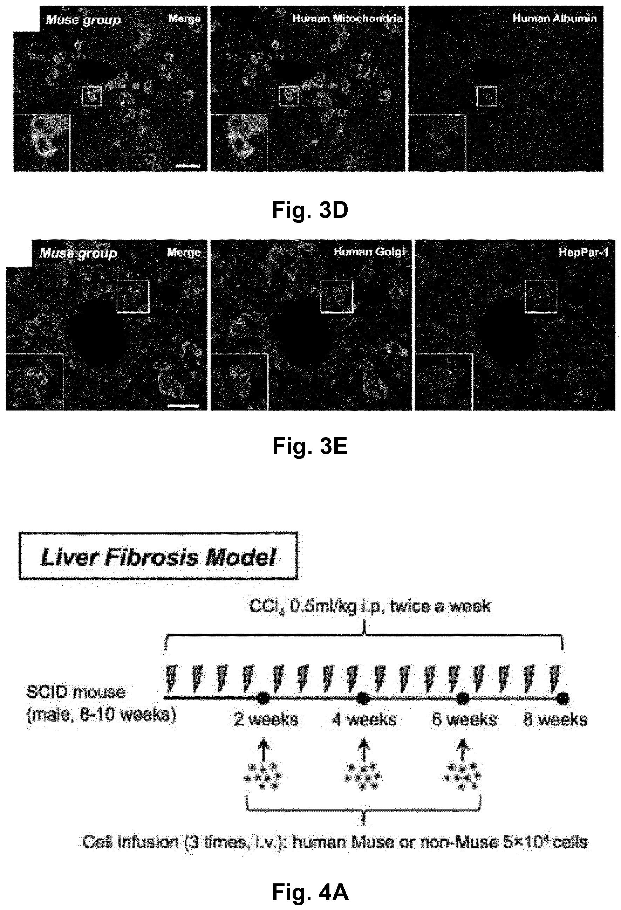

FIG. 3 shows results for *in vivo* dynamics of Muse cells and non-Muse cells in a liver injury model treated with CCl₄ for 24 hours. (A) Quantification results of human genome-specific Alu sequence present in liver two weeks after intravenous injection of human Muse or non-Muse cells in CCl₄-treated and intact groups. (B) Photomicrographs showing results of human Golgi (H-Golgi) immunostaining of liver at day 30 after cell injection (Golgi bodies in human cells are labeled, reflecting the localization of human Muse cells engrafted in mouse liver). (C) The proportions of H-Golgi (+) cell count to total cell count/ mm² in liver sections. ^{∗∗∗}: P < 0.001. (D, E) Photomicrographs showing results of double staining for H-Golgi/ human mitochondrion and hepatocyte marker HepPar-1, respectively. Scale bar: 50 µm.
FIG. 4 shows functional and histological evaluations using a liver fibrosis model. (A) Procedure for preparing a CCl₄-induced liver fibrosis model mouse, and administration regimen of Muse cells. (B, C) Serum total bilirubin (B) and serum albumin (C) concentrations in Muse group, vehicle group, and non-Muse group at week 8 after initiation of CCl₄ injection. (D, E) Photomicrographs showing evaluation of liver fibrosis areas with Sirius red staining (D) and Masson's trichrome staining (E). The graphs show numerical results of the areas. : P < 0.01, : P < 0.001. Scale bar: 50 µm.

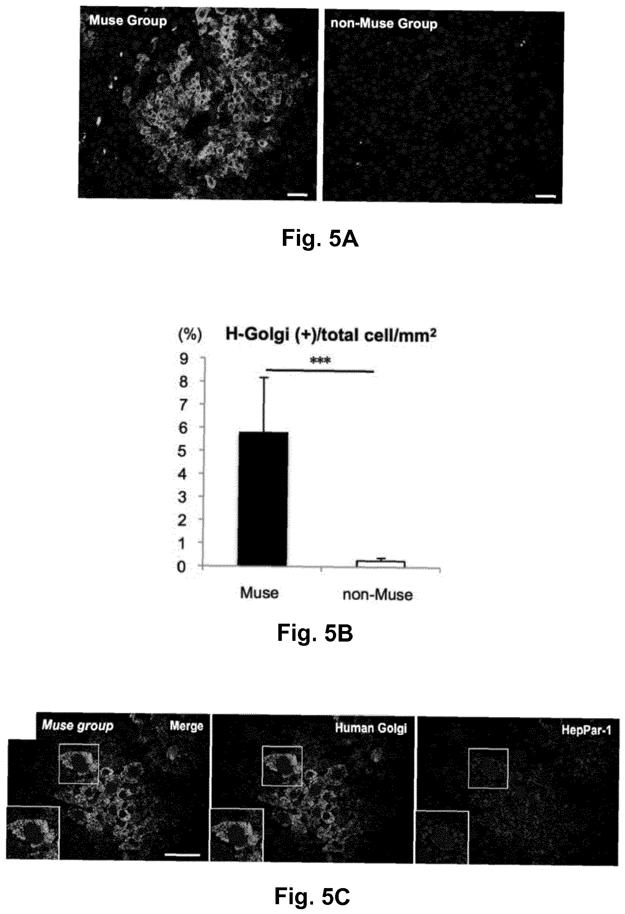

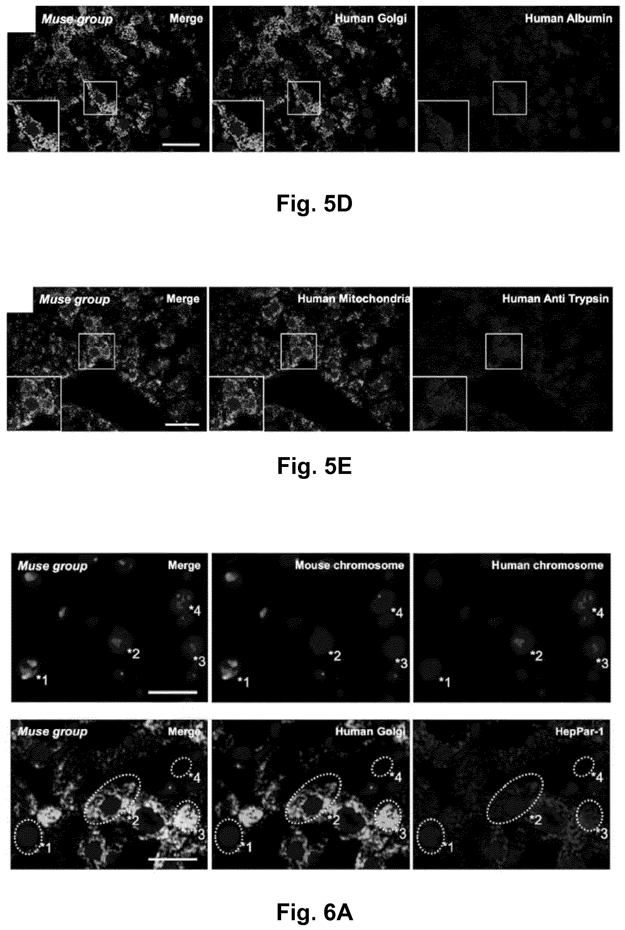

FIG. 5 shows results for differentiation of human Muse cell into liver tissue in a CCl₄-induced liver fibrosis model. (A) The number of H-Golgi (+) cells present in a liver at week 8 after initiation of CCl₄ treatment. In the Muse group, a considerable number of H-Golgi (+) cells were observed around blood vessels in the liver. On the other hand, in the non-Muse group, only a few of these cells were observed. (B) The proportions of H-Golgi (+) cell count to total cell count/ mm² in liver sections. ^{∗∗∗}: P < 0.001. (C) Immunostaining results (photomicrographs) showing the expression of HepPar-1 (a hepatocyte marker) in H-Golgi (+) cells. (D) Immunostaining results (photomicrographs) showing the expression of human albumin in H-Golgi (+) cells. (E) Immunostaining results (photomicrographs) showing the expression of human antitrypsin (a hepatocyte marker) in human mitochondrion (+) cells (representing human cell). Scale bar: 50 µm.

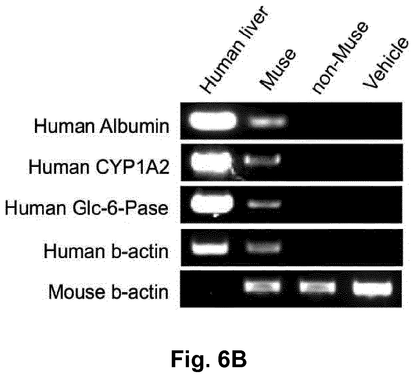

FIG. 6 shows results of FISH and functional analyses of a Muse group at week 8 after initiation of CCl₄ treatment. (A) The top panels show results of FISH, and the bottom panels show results (photomicrographs) of H-Golgi/HepPar-1 immunostaining in an adjacent section. In the FISH analysis, mouse chromosome was stained in green, while human chromosome was stained in red. In the results, the cell marked ^{∗}1 is assumed to be a mouse cell, the cells marked ^{∗}2 and ^{∗}3 are human cells that are not fused with mouse cells, and the cell marked ^{∗}4 is a human-mouse fused cell. Since the sections were prepared in 8 to 10 µm thickness, positions of nucleoli and cytoplasmic shapes do not exactly match between the adjacent sections. However, the cells marked 1, 2, and 3 in FISH can be overlapped with the corresponding cells in the immunostaining. The cell marked 4 was not overlapped with the immunostaining. The cell marked 1 is negative for H-Golgi. The cells marked ^{∗}2 and ^{∗}3 are double-positive for H-Golgi (+) and HepPar-1, suggesting that the H-Golgi (+)-human Muse cells were differentiated to HepPar-1 (hepatocyte marker) (+) cells without fusion with mouse hepatocytes. Scale bar: 25 µm. (B) Results of RT-PCR for human specific-albumin, -CYP1A2, -Glc-6-Pase, and human and mouse beta actin (photo). A human liver was used as a positive control, and a vehicle-treated liver group was used as a negative control.
FIG. 7 is a plot showing the amount of skin collagen in Control group, bleomycin (BLM)-28 day + Muse cell-treated group, and BLM-28 day + vehicle-treated group.
FIG. 8 is a plot showing the thickness of dermis layer in Control group, BLM-28 day + Muse cell-treated group, and BLM-28 day + vehicle treated group.
FIG. 9 shows results (photomicrograph, x 100) of hematoxylin-eosin staining of skin section from Control group, BLM-28 day + Muse cell-treated group, and BLM-28 day + vehicle-treated group. The double-headed arrow represents the thickness of the skin.
FIG. 10 shows fibrosis scores in left lobe and all lobes of lung from Control group and Muse cell-treated group.
FIG. 11 shows results (photomicrographs) of hematoxylin-eosin staining of lung section from Control group and Muse cell-treated group.
FIG. 12 is a graph showing changes in incidence of increased respiration rate with time in Control group and Muse cell-treated group.

### DETAILED DESCRIPTION OF THE INVENTION

The present invention relates to a cell product for prevention and/or treatment of organ fibrosis, the cell product comprising a SSEA-3-positive pluripotent stem cell (Muse cell). The present invention is described in detail below.

### 1. Indications

In the present invention, the cell product comprising a SSEA-3-positive pluripotent stem cell (Muse cell) provides prevention and/or treatment of organ fibrosis. The term "organ fibrosis" refers to any disease in which fibrosis occurs in organs and/or tissues by various causes such as infection, inflammation, accumulation of endogenous substances such as fat, and denaturation of tissues and cells, caused by various causes such as microorganisms, chemicals, *in vivo* responses such as immune response, food habits, environment, and inheritance (including an unknown cause), impairing the function of the organs and/or tissues. Examples of the organ fibrosis include fibrosis occurring in liver (liver fibrosis), pancreas (pancreatic fibrosis), and digestive organs such as large intestine; fibrosis occurring in respiratory organs such as lung (pulmonary fibrosis); fibrosis occurring in cardiovascular organs such as heart (myocardial fibrosis), bone marrow (myelofibrosis), and spleen; fibrosis occurring in urogenital organs such as kidney (kidney fibrosis); fibrosis occurring in locomotor organs such as muscle; and various fibrosis occurring in central nervous system, endocrine organs, skin, etc.

### 2. Cell product

### (1) Pluripotent Stem Cell (Muse Cell)

The pluripotent stem cell used in the cell product of the present invention is a cell that was found in human living body and named "Muse (Multilineage-differentiating Stress Enduring) cell" discovered by Dezawa, one of the present inventors. It is known that Muse cells can be obtained from, for example, bone marrow aspirate, adipose tissue (Ogura, F., et al., Stem Cells Dev., Nov 20, 2013 (Epub) (published on Jan 17, 2014)) and dermal connective tissue of skin, and also are broadly present in tissues and organs. This cell also has both characteristics of pluripotent stem cell and mesenchymal stem cell and is identified as, for example, a cell positive for "SSEA-3 (Stage-specific embryonic antigen-3)," a cell surface marker, preferably as a double-positive cell that is positive for SSEA-3 and CD-105. Therefore, Muse cells or a cell fraction containing Muse cells can be isolated from living tissues using, for example, expression of SSEA-3 only or a combination of SSEA-3 and CD-105 as cell surface marker. Methods for separation and identification of, and characteristics of Muse cell have been specifically disclosed in WO2011/007900. Muse cells can also be selectively enriched by utilizing the high resistance of Muse cells to various external stresses and culturing under various external stress conditions, such as under protease treatment, under hypoxic condition, under low-phosphate condition, in a low serum concentration, under low-nutrition condition, under heat shock exposure, in the presence of toxic substance, in the presence of reactive oxygen species, under mechanical stimulation, and under pressure treatment. As used herein, the pluripotent stem cells (Muse cells) or a cell fraction containing Muse cells prepared, as a cell product for treating fibrosis, from mesenchymal tissues or cultured mesenchymal tissues using SSEA-3 as cell surface marker may be simply referred to as "SSEA-3-positive cells." As used herein, the term "non-Muse cells" may refer to cells contained in mesenchymal tissues or cultured mesenchymal tissues and excluding "SSEA-3-positive cells."

Muse cells or a cell fraction containing Muse cells can be prepared from living tissues (e.g., mesenchymal tissues) using cell surface markers, SSEA-3 or SSEA-3 and CD-105, as cell surface marker. As used herein, the term "living" means mammal living body. In the present invention, the living body does not include fertilized egg and embryos in developmental stages before blastocyst stage, but includes embryos in developmental stages of blastocyst stage or later, including fetus and blastula. Examples of the mammal include, but not limited to, primates such as human and monkey; rodents such as mouse, rat, rabbit, and guinea pig; and cat, dog, sheep, pig, cattle, horse, donkey, goat, and ferret. The Muse cell used in the cell product of the present invention is definitively distinguished from embryonic stem cells (ES cells) and iPS cells in that the Muse cell are directly isolated with markers from living tissues. The term "mesenchymal tissue" refers to tissues present in tissues or various organs such as bone, synovial membrane, fat, blood, bone marrow, skeletal muscle, dermis, ligament, tendon, dental pulp, umbilical cord, cord blood, and amnion. The Muse cells can be obtained from, for example, bone marrow, skin, adipose tissue, blood, dental pulp, umbilical cord, cord blood, and amnion. Preferably, a mesenchymal tissue of the living body is collected, and then Muse cells are prepared from the tissue and used. Alternatively, using the preparation method described above, the Muse cells may be prepared from cultured mesenchymal cells such as fibroblast and bone marrow mesenchymal stem cell.

The cell fraction containing Muse cells used in the cell product of the present invention can also be prepared by a method comprising exposure of mesenchymal tissues of the living body or cultured mesenchymal cells to an external stress in order to selectively allow stress-tolerant cells to proliferate and collection of the cells with the increased abundance ratio of stress-tolerant cells.

Above-mentioned external stress may be any of the following: protease treatment, culture under hypoxia, culture under low-phosphate condition, culture under low serum concentration, culture undernutrition condition, culture under heat shock exposure, culture at low temperatures, freezing treatment, culture in the presence of toxic substances, culture in the presence of reactive oxygen species, culture under mechanical stress, culture under shaking, culture under pressure treatment or physical shocks, or combination thereof.

Above-mentioned protease treatment is preferably carried out for 0.5 to 36 hours in total to exert the external stress. The concentration of the protease may be a concentration used when cells adhered to a culture vessel are harvested, when cell aggregates are separated into single cells, or when single cells are collected from a tissue.

Preferably, above-mentioned protease is serine protease, aspartic protease, cysteine protease, metalloprotease, glutamic protease or N-terminal threonine protease. More preferably, above-mentioned protease is trypsin, collagenase or Dispase.

The Muse cell used in the cell product of the present invention may be autologous or allogeneic to a recipient of cell transplantation.

As described above, Muse cells or a cell fraction containing Muse cells can be prepared from tissues of the living body, for example, by using SSEA-3-positivity or SSEA-3 and CD-105-double-positivity as cell surface marker. Human adult skin is known to comprise various types of stem cells and precursor cells. However, Muse cell is different from these cells. These stem cells and precursor cells include skin-derived precursor cell (SKP), neural crest stem cell (NCSC), melanoblast (MB), pericyte (PC), endothelial precursor cell (EP), and adipose-derived stem cell (ADSC). Muse cells can be prepared using "non-expression" of markers unique to these cells as cell surface marker. More specifically, Muse cells can be isolated using as an index of negative expression for at least one, e.g., 2, 3, 4, 5, 6, 7, 8, 9, 10 or 11, of 11 cell surface markers selected from the group consisting of CD34 (a marker for EP and ADSC), CD117 (c-kit) (a marker for MB), CD146 (a marker for PC and ADSC), CD271 (NGFR) (a marker for NCSC), NG2 (a marker for PC), vWF factor (von Willebrand factor) (a marker for EP), Sox10 (a marker for NCSC), Snai1 (a marker for SKP), Slug (a marker for SKP), Tyrp1 (a marker for MB), and Dct (a marker for MB). Muse cells can be prepared by using as an index of negative expression for, for example, but not limited to, CD117 and CD146; CD117, CD146, NG2, CD34, vWF and CD271; or the above-described 11 markers.

The Muse cell having the above-described characteristics and used in the cell product of the present invention also has at least one selected from the group consisting of the following characteristics:
(i) having low or no telomerase activity;
(ii) capable of differentiating into any of tridermic cells;
(iii) showing no neoplastic proliferation; and
(iv) having self-renewal capacities.
Preferably, the Muse cell used in the cell product of the present invention has all of the characteristics described above. With respect to (i) above, the phrase "having low or no telomerase activity" means that the telomerase activity is low or undetectable when detected using, for example, TRAPEZE XL telomerase detection kit (Millipore). Having "low" telomerase activity means, for example, having a telomerase activity comparable to somatic human fibroblast, or having 1/5 or less telomerase activity, preferably one-tenth or less telomerase activity, as compared with that of HeLa cell. With respect to (ii) above, the Muse cell is capable of differentiating into triploblastic cells (endodermal, mesodermal, and ectodermal cells) *in vitro* and *in vivo.* For example, the Muse cell can differentiate into hepatocyte (including cells expressing hepatoblast markers or hepatocyte markers), neuron, skeletal muscle cell, smooth muscle cell, osteocyte, or adipocyte by *in vitro* culture for induction. The Muse cell may also be able to differentiate into triploblastic cells when it is transplanted in testis *in vivo.* Further, the Muse cell is capable of migration and engraftment into injured organs (such as heart, skin, spinal cord, liver, and muscle) and differentiation into cells suitable for the tissues when transplanted to a living body via intravenous injection. With respect to (iii) above, in suspension culture, the Muse cells can proliferate from single cell at a growth rate of about 1.3 days in suspension culture and form cell clusters similar to embryoid body and then arrest their proliferation after about 14 days. When these cell clusters similar to embryoid body are transferred to adherent culture, the cells restart proliferation and cells proliferated from the cell clusters spread. Further, the cells are characterized in that, when transplanted into testis, they do not become cancerous for at least half a year. With respect to (iv) above, the Muse cell has self-renewal (self-replication) capacities. The term "self-renewal" means that differentiation into three-germ layer cells from cells contained in the first cell clusters similar to embryoid-body derived by single Muse cell in a suspension culture can be observed; that formation of the second-generation of embryoid-body-like clusters by again culturing single cell of the first-generation of embryoid-body-like clusters in a suspension culture can be observed; and that differentiation into three-germ layer cells and formation of the third-generation of embryoid-body-like clusters obtained by single-cell suspension culture derived from the second-generation of embryoid-body-like clusters can be observed. Self-renewal means to be able to repeat for one or more above-mentioned experimental cycles.

### (2) Preparation and Use of Cell product

The method of obtaining the cell product of the present invention include, but not limited to, suspending Muse cells or a cell fraction containing Muse cells obtained in (1) above in a physiologic saline or a suitable buffer solution (e.g., phosphate buffered saline). In this case, if only small numbers of Muse cells are obtained from an autologous or allogeneic tissue, these cells may be cultured before cell transplantation until the fixed number of cells is obtained. As previously reported (WO2011/007900), since Muse cells do not become tumorigenic, if cells collected from a living tissue and some undifferentiated cells remain, they have low possibility of converting to malignant cells and thus are safe. The collected Muse cells can be cultured in any common culture medium (e.g., α-minimum essential medium (α-MEM) supplemented by 10% calf serum). More specifically, with reference to the above-described WO2011/007900, for example, a culture medium, and additives (e.g., antibiotics, and serum) are appropriately selected for culture and proliferation of Muse cells, so that a solution containing the fixed concentration of Muse cells can be prepared. When the cell product of the present invention is administered to human subject, bone marrow aspirates are collected from a human ilium, and then, for example, bone marrow mesenchymal stem cells are cultured to obtain as adherent cells from the bone marrow aspirate and proliferated until reaching the cell amount where a therapeutically effective amount of Muse cells can be obtained. Thereafter, Muse cells are sorted using an antigenic marker SSEA-3 as cell surface marker. These autologous or allogeneic Muse cells can be used for preparing the cell product. Alternatively, for example, bone marrow mesenchymal stem cells obtained from the bone marrow aspirates are cultured under external stress conditions to proliferate and enrich Muse cells until they reach a therapeutically effective amount. Then, these autologous or allogeneic Muse cells can be used for preparing the cell product.

When the Muse cells are used in the cell product, the cell product may contain dimethyl sulfoxide (DMSO), serum albumin or the like for protection of the cells and antibiotics or the like for prevention of contamination and proliferation of bacteria. The cell product may further contain other pharmaceutically acceptable components (e.g., carrier, excipient, disintegrant, buffer agent, emulsifier, suspending agent, soothing agent, stabilizer, preservative, antiseptic, physiologic saline), or cells or components other than Muse cell contained in the mesenchymal stem cells. These agents and drugs can be added to the cell product in an appropriate concentration by the skilled person. Thus, Muse cells can also be used as a pharmaceutical composition containing various additives.

The number of Muse cells contained in the cell product prepared above can be appropriately adjusted to obtain desired effects (e.g., for liver fibrosis, recovery of serum total bilirubin and albumin, and reduction in fibrosis) in treatment of fibrosis, in consideration of, for example, sex, age, and weight of subjects, condition of diseased part, and condition of cells to be used. Individuals to be the subject includes, but not limited to, human. The cell product of the present invention may be administered multiple times (e.g., 2 to 10 times) at appropriate intervals (e.g., twice a day, once a day, twice a week, once a week, once every two weeks, once a month, once every two months, once every three months, or once every six months) until a desired therapeutic effect is obtained. Thus, depending on the condition of the subject, the therapeutically effective amount preferably is a dosage of, for example, 1×10³ to 1 ×10⁸ cells/individual/dose in 1 to 10 doses. Examples of total dosage for an individual include, but not limited to, 1×10³ to 1×10⁸ cells, 1×10⁴ to 5×10⁷ cells, 2×10⁴ to 2×10⁷ cells, 5×10⁴ to 5×10⁶ cells, and 1×10⁵ to 1×10⁹ cells.

The Muse cell used in the cell product of the present invention is characterized by migration and engraftment to injured organs. Thus, in regard to the administration of the cell product, the administration route of the cell product, and the type of the blood vessel into which the cell product is administered (vein or artery) are not limited.

The cell product of the present invention can improve or restore the function of injured organs in patients with fibrosis to normal (or normal levels). As used herein, the term "improvement" of organ function means relief and inhibition of progression of various symptoms associated with fibrosis, preferably relief of the symptoms to the extent that they do not interfere with daily life. As used herein, the term "restore to normal" organ functions means that all symptoms caused by fibrosis are restored to the states before the organ injury.

In the case of liver fibrosis, for example, the function of a liver after administration of the cell product of the present invention can be evaluated by, for example, determination of serum total bilirubin level or albumin level, or expression of liver marker gene.

The present invention will be described in detail with reference to examples below, but is not limited to the examples.

### EXAMPLES

### Materials and Methods

### Preparation of Human Muse Cell

Human Muse cells were prepared according to the method described in WO2011/007900. More specifically, the Muse cells used in Example 1 were obtained by culturing mesenchymal cells having adhesive property from human bone marrow aspirates; allowing the cells to proliferate; and then sorting Muse cells or a cell fraction containing Muse cells as SSEA-3-positive cells by FACS. The Muse cells used in Examples 2 to 4 were obtained by culturing mesenchymal stem cells under stress conditions for expansive enrichment culture. Non-Muse cells were a sub-population of SSEA-3-negative cells of the above-described mesenchymal cells, and used as a control. The cells were then adjusted into a fixed cell density using a phosphate buffered saline or a culture medium and used for the following experiments.

### Example 1

### Transplantation of Human Muse Cell in Mouse Model with Liver Fibrosis

CB17/Icr-Prkdc<scid>/CrlCrlj (SCID) mice were used in all studies. All animal experiments were conducted according to the guideline of the Animal Care and Experimentation Committee of Tohoku University (Sendai, Japan). With respect to experimental procedures for establishing the liver fibrosis model, see also International Journal of Molecular Science 2012; 13: 3598-3617 and Journal of Biochemistry 2003; 134: 551-558. Specifically, an SCID mouse model with liver fibrosis was produced by intraperitoneal injection of CCl₄ (0.5 ml/kg).

Human Muse cells or non-Muse cells (5×10⁴ cells) were injected into the tail vein of liver fibrosis model mice.

### Statistical Analysis

Significant differences between two groups were evaluated using Student's t test. Statistical significant differences among three or more groups were evaluated using one-way analysis of variance (one-way ANOVA) with Bonferroni's multiple comparison test. P < 0.05 (in Figures, shown with ^{∗}) was defined as significant, while <0.01 (^{∗∗}) or <0.001 (^{∗∗∗}) was defined as highly significant.

### Results

### Characterization of Muse cell

SSEA-3-positive human Muse cells (about 2% of human BM-MSCs) and SSEA-3-negative non-Muse cells (control group) were sorted by FACS (FIG. 1A). When these cells were cultured in single-cell suspension culture, only the Muse cells produced single cell-derived clusters (M-clusters) similar to ES cell-derived embryoid body formed in suspension culture, while, the non-Muse cells did not form such cluster at all (data not shown). Expressions of pluripotency marker genes in adherent-cultured Muse cells, adherent-cultured non-Muse cells, and M-clusters were investigated. It was demonstrated that the adherent-cultured Muse cells showed higher gene expression of pluripotency markers, OCT4, SOX2, and Nanog, as compared with the adherent-cultured non-Muse cells. In addition, SOX2 and Nanog expressions in the adherent-cultured non-Muse cells were below detection threshold. In particular, expression levels of OCT4, SOX2, and Nanog in M-clusters were about 9-times, about 54-times, and about 35-times higher, respectively, than those of the adherent-cultured Muse cells, showing statistically significant differences (FIG. 1B). Triploblastic differentiation ability (differentiation into triploblastic lineage cells) of single Muse cell was confirmed as described in Proc Natl Acad Sci USA 2010; 107: 8639-8643. It has already been reported that Muse cells differentiate to alpha-fetoprotein/albumin-positive cells induced by hepatocyte growth factor (HGF) and fibroblast growth factor 4 (FGF-4) (Proc Natl Acad Sci USA 2011; 108: 9875-9880). Thus, whether cells proliferated from M-clusters formed in single-cell suspension culture on a gelatin-coated culture dish (FIG. 1C) spontaneously differentiated into cells expressing hepatoblast and hepatocyte markers was determined. This revealed that these cells comprised cells positive for DLK (1.5 ± 0.6%), alpha-fetoprotein (3.0 ± 0.8%), cytokeratin 19 (1.7 ± 0.4%), or cytokeratin 18 (2.0 ± 0.9%) (FIG. 1D). Since non-Muse cells do not form clusters in single-cell suspension culture, they were directly plated in a gelatin-coated culture dish soon after isolation thereof and cultured for the same length of time as M-cluster. However, the non-Muse cells did not show any expression of hepatoblast or hepatocyte lineage markers (data not shown). Therefore, it is assumed that Muse cells are high differentiation ability for hepatoblast and hepatocyte lineage cells, while non-Muse cells do not have such ability.

### Muse Cells Efficiently Migrate and Engraft to an Injured Liver

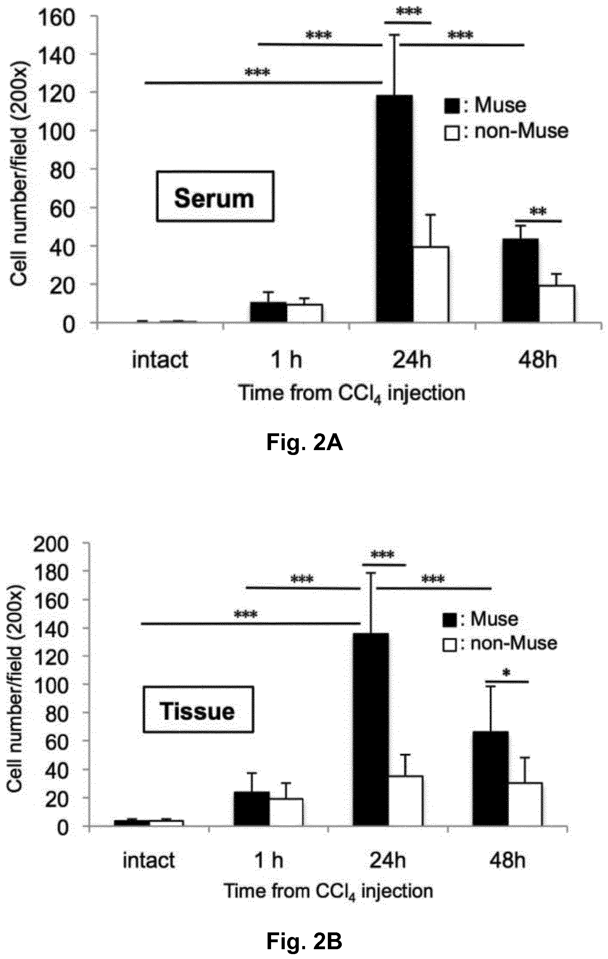

Next, the abilities of human Muse cells to migrate to serum and a liver tissue in a liver injury model were investigated *in vitro.* For this, a single dose of carbon tetrachloride (CCl₄) was intraperitoneally injected to immunodeficient mice (SCID mice) that did not reject human cells, and then their sera and injured liver tissues were collected at 1, 24, and 48 hours after the injection. As controls, sera and liver tissues in SCID mice that were not administered with carbon tetrachloride (CCl₄) were collected. The Boyden chamber assay was used to evaluate the migration ability. Collected sera or tissues were placed into the lower side of an insert, and human Muse cells or non-Muse cells were placed into the upper side of the insert. Then, the number of human Muse cells or non-Muse cells that passed through the insert was determined. As a result, in the sera from intact mice, only a few Muse and non-Muse cells were observed to migrate, and there was no statistical difference between them (FIG. 2A). In the sera obtained at 1 hour after CCl₄ injection (1 hr-CCl₄), number of both migrating Muse and non-Muse cells were slightly increased, but there was no statistical difference between intact and 1 hr-CCl₄ sera for both Muse and non-Muse cells. However, in the 24 hrs-CCl₄ sera, the number of Muse cells that migrated to the sera greatly increased, showing statistically significant differences as compared with those in the intact and 1 hr-CCl₄ sera (both p < 0.001). The increased number of Muse cells that have migrated at 24 hours was about 12 times greater than that in the 1 hr-CCl₄ serum. On the other hand, no such dramatic change was observed for non-Muse cells. At 24 hours, Muse cells showed 3 to 4 times higher migration rate than non-Muse cell, and a statistically significant difference was observed between them (p < 0.001). In 48 hrs-CCl₄ sera, though it was not at the level as seen in 24 hrs-CCl₄ sera, statistical difference was still observed between Muse cells and non-Muse cells (P < 0.01) (FIG. 2A).

As in the case of serum, the number of migrating Muse cells was the highest in 24 hrs-CCl₄ liver tissues, showing statistically significant differences as compared with that of non-Muse cells (p < 0.001), or as compared with that of Muse cells in intact, 1 hr-CCl₄, or 48 hrs-CCl₄ liver tissue (each p < 0.001). The number of migrating Muse cells in the 24 hrs-CCl₄ liver was about 7 times that of Muse cells in the 1 hr-CCl₄ liver, and about 4 times that of non-Muse cells in the 24 hrs-CCl₄ liver. When using non-Muse cells, no significant migration was observed (FIG. 2B). These results demonstrated that unlike non-Muse cell, Muse cells showed strong migration activity to serum and liver of the CCl₄-liver injury model.

The *in vivo* dynamic analyses for human Muse and non-Muse cells injected via tail vein were performed. Using SCID mice, intact mice and mice 24 hours after single intraperitoneal injection of carbon tetrachloride (CCl₄) (24 hrs-CCl₄-liver injury mice) were prepared, and then injected with human Muse cells or non-Muse cells via their tail veins. For organs from intact mice and 24 hrs-CCl₄-liver injury mice at week 2 after the administration of Muse cells, Q-PCR for human specific Alu sequence was carried out to investigate the distributions of human Muse cells and non-Muse cells. In the intact mice, in both Muse cell-injected and non-Muse cell-injected mice, low signals of Alu sequence were detected in lungs, while signals from the other organs were below detection limit (FIG. 3A). In the 24 hrs-CCl₄-liver injury model injected with Muse cells, signals were highest in liver, lower in lung, and below detection limit in the other organs. On the other hand, in the mice injected with non-Muse cells, signals were below detection threshold in liver and in the other organs other than lung (FIG. 3A).

Next, engraftment and differentiation of human Muse cells at day 30 after cell administration to a liver injury model (24 hrs-CCl₄-liver injury mouse) were investigated using an anti-human Golgi complex (H-Golgi) antibody or an anti-human mitochondrion antibody. Since tissue images of an injured liver were highly inhomogeneous, ten different regions were randomly selected from both injured regions and normal-appearing regions, and measurement was performed for all of them. The number of H-Golgi (+) cells detected in the Muse group (1.89 ± 0.65% of total cells in 1 mm² in liver sections) was about 48 times that in the non-Muse group (0.04 ± 0.08%), showing highly and statistically significant difference (p < 0.001) (FIGS. 3B and 3C). Histological analysis showed that H-Golgi (+) cells were mainly distributed around blood vessels in the liver in the Muse group, suggesting that intravenous injected Muse cells integrated into the liver from the blood vessels (FIG. 3B). Integration of human Muse cells into an injured liver was also confirmed by detection of human-specific mitochondria (FIG. 3D). Double staining with H-Golgi/human mitochondrion and hepatocyte markers demonstrated that 49.8 ± 1.9% of human mitochondrion (+) cells were positive for human-specific albumin, and that 80.4 ± 3.2% of H-Golgi (+) cells were positive for human progenitor/mature liver cell marker HepPar-1 (FIGS. 3D and 3E).

All these results showed that Muse cells had a much higher capacity for migration to and accumulation in the injured liver both *in vitro* and *in vivo,* and for differentiation into human specific albumin-positive and HepPar-1-positive cells *in vivo,* while non-Muse cells did not show such an ability.

### Muse Cells Improve Functions and Attenuate Fibrosis in Liver Fibrosis Model

The experimental procedure for preparing the liver fibrosis model as well as numbers of cells injected and timing of injection are shown in FIG. 4A. Specifically, the SCID mouse model of liver fibrosis was established by carrying out intraperitoneal injection of CCl₄ (0.5 ml/kg) twice a week for up to 8 weeks.

To the liver fibrosis model mice, 5×10⁴ human Muse cells (Muse group, n = 8) or non-Muse cells (non-Muse group, n = 8), or equivalent volume of phosphate buffered saline (PBS) (vehicle group; n = 8) were injected via their tail vein at weeks 2, 4, and 6, and then data were collected at week 8.

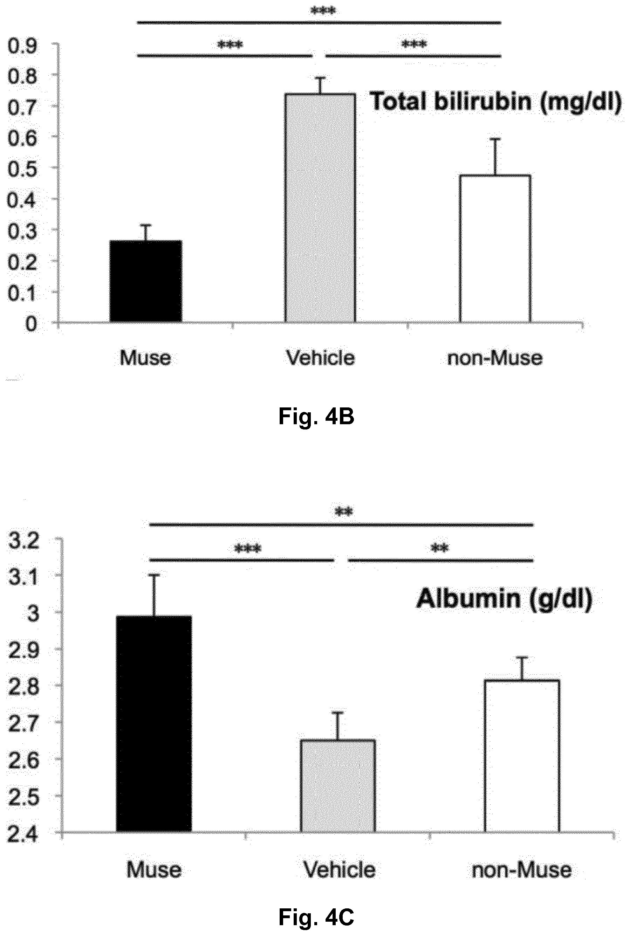

In the Muse, non-Muse, and vehicle groups, no tumorigenesis was observed up to week 8 (data not shown). At week 8, serum total bilirubin (0.26 ± 0.05 mg/dl) was significantly lower in the Muse group than those in the vehicle group (0.74 ± 0.05 mg/dl, p < 0.001) and the non-Muse group (0.48 ± 0.12 mg/dl, p < 0.001), and the value in the Muse group was about 2.8 times lower than that in the vehicle group. Although not as much as the decrease observed in the Muse group, total bilirubin of the non-Muse group was 1.5 times lower than that of the vehicle group, showing statistically significant difference (p < 0.001). This suggested a moderate recovery occurred (FIG. 4B). The serum albumin concentration in the Muse group was the highest among the 3 groups (2.99 ± 0.11 g/dl), showing highly and statistically significant differences as compared with those of the vehicle group (2.65 ± 0.08 g/dl, p < 0.001) and the non-Muse group (2.81 ± 0.06 g/dl, p < 0.01). Although not as much as the increase observed in the Muse group, the non-Muse group showed a restoration of serum albumin at a moderate level as compared with that in the vehicle group (p < 0.01) (FIG. 4C).

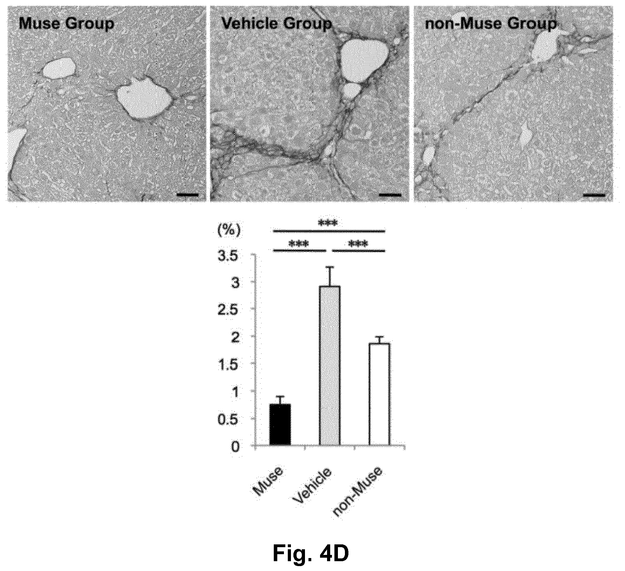

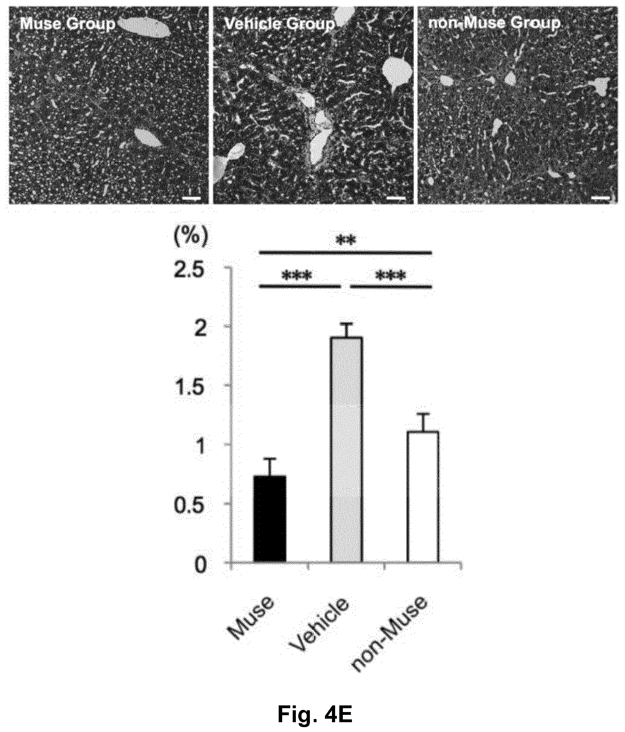

The extent of fibrosis mainly composed of type I/III collagen was evaluated by Sirius red staining and Masson's trichrome staining. At 8 weeks, a widespread of the fibrotic area with typical internodular septum was observed in the vehicle group. On the other hand, the fibrotic area was the smallest in the Muse group. The Sirius red staining revealed that the Muse group showed the smallest fibrotic area (0.75 ± 0.15% of the total area per section) as compared with those in the vehicle group (2.91 ± 0.35%) and the non-Muse group (1.86 ± 0.13%), both with statistically significant differences (p < 0.001), improving fibrosis by 75% compared to the vehicle group (FIG. 4D). A statistical difference was observed between the non-Muse group and the vehicle group (p < 0.001), and the fibrosis in the non-Muse group corresponded to 36% improvement compared to the vehicle group, suggesting a moderate effect in the non-Muse group (FIG. 4D). Similar results were obtained in the Masson's trichrome staining. The Muse group exhibited the lowest fibrotic area among the 3 groups (0.73 ± 0.15%) with highly statistically significant differences as compared with those of the vehicle group (1.90 ± 0.12%, p < 0.001) and the non-Muse group (1.11 ± 0.15%, p < 0.01), improving fibrosis by 62% compared to the vehicle group (FIG. 4E). Although not as much as the decrease observed in the Muse group, the non-Muse group showed significant difference (p < 0.001) as compared with the vehicle group, which corresponded to a 42% improvement compared to the vehicle group (FIG. 4E).

These results showed that liver function measured with serum total bilirubin and albumin was improved, and fibrosis in a liver fibrosis model mouse up to week 8 was attenuated more effectively in the human Muse group than in the non-Muse group.

### Muse Cells Provide New Hepatocytes through In Vivo Spontaneous Differentiation in a Liver Fibrosis Model

At week 8, a large number of human Muse cells were detected in area around the vessels, while only a few non-Muse cells were detected (FIG. 5A). The Muse group demonstrated a higher percentage of H-Golgi (+) cells per total cells in 1-mm² section (5.78 ± 2.39%), while that in the non-Muse group was extremely lower in the non-Muse group (0.27 ± 0.12%), with a statistically significant difference (p < 0.001), representing about 21 times higher numbers of H-Golgi cells in the Muse group (FIGS. 5A and 5B).

Immunohistochemistry was further performed in the Muse group. Muse cells that were positive for H-Golgi and human mitochondrion were detected in a liver, expressing HepPar-1 (71.1 ± 15.2% of H-Golgi (+) cells) (FIG. 5C), human albumin (54.3 ± 8.2% of H-Golgi (+) cells) (FIG. 5D), and human antitrypsin (47.9 ± 4.6% of human mitochondrion (+) cells) (FIG. 5E). Therefore, integrated human Muse cells are suggested to differentiate spontaneously into hepatocyte marker-positive cells after integration.

Previous studies have posited that bone marrow-derived hepatocytes in the injured liver may be occasionally formed by cell fusion. In order to investigate whether the above-mentioned differentiation in this study was a result of cell fusion or not, fluorescence *in situ* hybridization (FISH) analysis was performed to investigate the existence of cell fusion between host hepatocytes (derived from mouse, colored in green) and injected Muse cells (derived from human, colored in red) (FIG. 6A). Neighboring sections of each FISH sample were subjected to double staining of H-Golgi and HepPar-1 to determine whether FISH signals could be derived from differentiated human Muse cells. As a result, only 2.6 ± 0.2% of H-Golgi (+)/HepPar-1 (+)-human Muse cells that would approximately matching with the cells in the FISH analysis were suggested to be generated by cell fusion. This suggested that about 97% of human Muse cells incorporated into the mouse liver tissue and differentiated into hepatocyte marker-positive cells without cell fusion.

Expressions of human-specific mature functional hepatocyte markers, such as human-specific albumin, human cytochrome P450 1A2 (CYP1A2), an enzyme involved in drug metabolism, and human glucose-6-phosphatase (Glc-6-Pase), an enzyme related to free glucose, were investigated by RT-PCR, and their high level of expressions were observed in a liver in the Muse group. On the other hand, in the non-Muse group and the vehicle group, these markers were not expressed. Remarkably, human beta actin was below detection level in the non-Muse-transplanted liver (FIG. 6B). This result is consistent with the histological data of the non-Muse group in FIGS. 5A and 5B.

One possible mechanism of fibrosis improvement was that proteases such as metalloprotease lysed fibers such as collagen.

From the above, Muse cells showed differentiation ability into hepatoblast/hepatocyte lineage cells *in vitro.* Muse cells showed strong ability to migrate towards sera and livers in mice treated with CCl₄ *in vitro.* Muse cells also specifically accumulated in injured livers *in vivo,* while they did not show specific accumulation in other organs. Muse cells after engraftment in a liver spontaneously differentiated to cells positive for HepPar-1 (71.1 ± 15.2%), human albumin (54.3 ± 8.2%), and antitrypsin (47.9 ± 4.6%) *in vivo* without fusion with host hepatocytes, and expressed mature and functional human markers such as CYP1A2 and Glc-6-Pase at week 8. Further, substantial restoration of serum total bilirubin and albumin, and attenuation in fibrosis were observed.

These results suggest that Muse cells are effective in preventing and treating liver fibrosis, as well as effective in preventing and treating other fibrosis.

### Example 2

### Evaluation of Muse Cells in a Skin Fibrosis Model

BLM-induced skin fibrosis mouse model was prepared according to the method described in Sci Rep. 2015 Aug 20; 5: 12466. doi: 10.1038/srep12466. Female C57BL/6J mice (Japan SLC, Inc.) were used. For control group, physiologic saline was administered instead of BLM. At day 14 after the administration of BLM, 1×10⁶ cells/weight (kg) of Muse cells were administered via their tail veins. On the other hand, for vehicle-treated group, a vehicle was administered via their tail veins at day 14.

Skin tissues were isolated at day 28 after the administration of BLM and subjected to collagen quantification, hematoxylin-eosin (HE) staining, and dermal thickness analysis.

The skin collagen analysis was performed as described below.

Skin sections were homogenized with 0.5 M acetic acid/pepsin solution, and stirred overnight at 4°C. The concentration of skin collagen in the obtained extract was measured by using Sircol collagen assay kit (Biocolor, Cat No: S1000).

For the HE staining and dermal thickness analysis, sections of skin (4 µm) were first prepared and then subjected to HE staining, and photographs of five fields per section were taken at magnification of 100x under a light microscope. The distance from just under the epidermal layer to the subcutaneous fat was dermal thickness and measured using Image J.

### Analysis of Plasma Hyaluronic Acid

Using plasmas obtained from the mice, the concentration of hyaluronic acid in the plasma was determined using ELISA (Quantikine Hyaluronan ELISA Kit, R&D systems, Inc.).

### Results

### 1. Skin Collagen Content

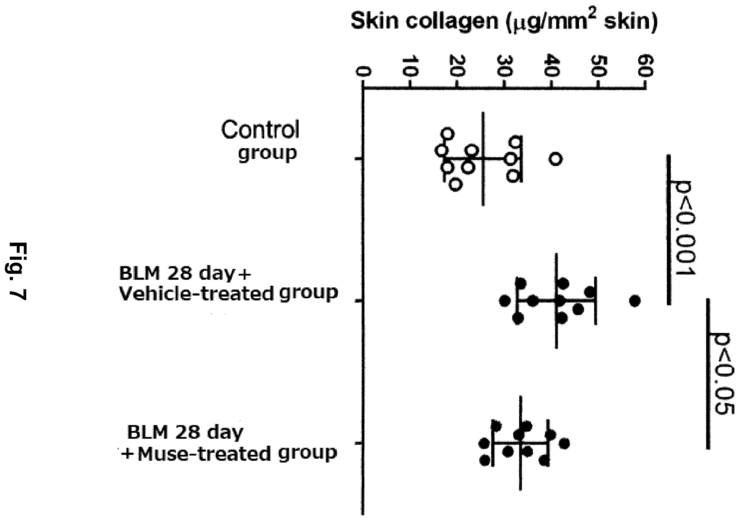

The results obtained from the analysis of skin collagen content are shown in FIG. 7. A significant increase in skin collagen content was observed in the BLM-28 day + vehicle-treated group as compared with the control group not receiving BLM. A significant decrease in skin collagen content was observed in the BLM-28 day + Muse cell-treated group as compared with the BLM-28 day + vehicle-treated group.

### 2. HE Staining and Dermal Thickness

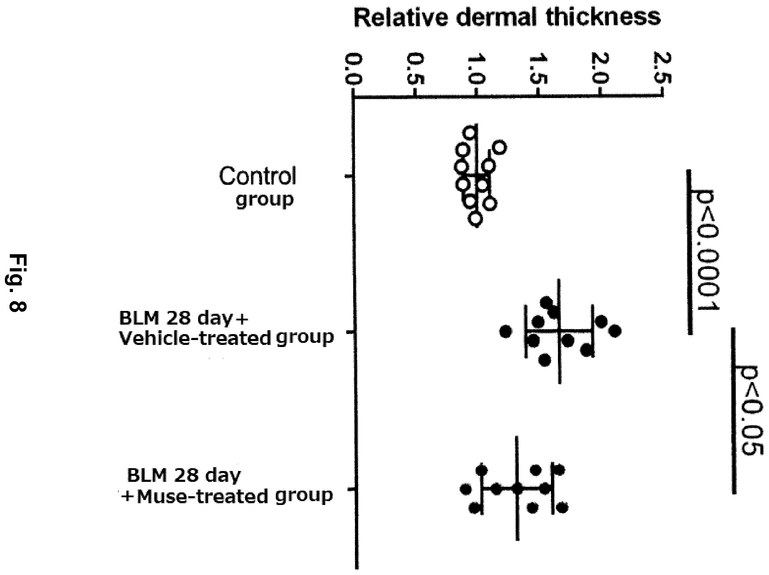

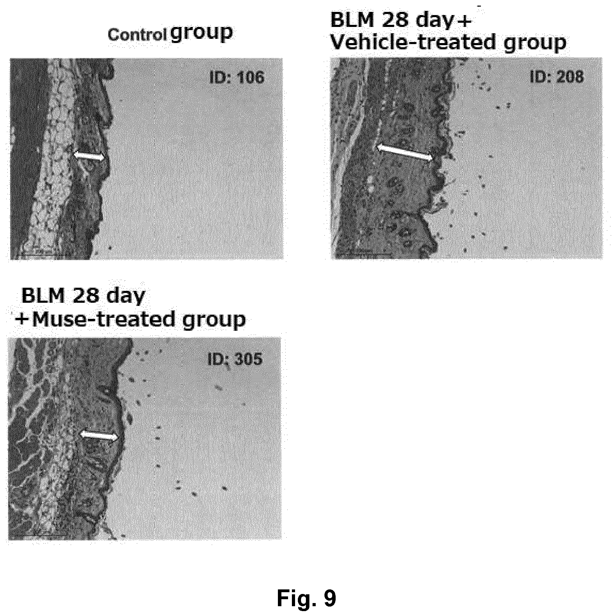

The HE staining images are shown in FIG. 9, and the dermal thicknesses are shown in FIG. 8. A significant increase in dermal thickness was observed in the BLM-28 day + vehicle-treated group as compared with the control group. A significant decrease in dermal thickness was observed in the BLM-28 day + Muse cell-treated group as compared with the BLM-28 day + vehicle-treated group.

### 3. Plasma Hyaluronic Acid Concentration

With respect to plasma hyaluronic acid concentration, significant difference between the control group and the BLM-28 day + vehicle-treated group was not shown. On the other hand, the plasma hyaluronic acid concentration in the BLM-28 day + Muse cell-treated group tended to increase as compared with the BLM-28 day + vehicle-treated group.

### Example 3

### Evaluation of Muse Cells in a Pulmonary Fibrosis Model

BLM pulmonary fibrosis model mice were prepared by intratracheal administration of BLM solution (14 µg/25 µL/lung) (Japanese Journal of Medicine and Pharmaceutical Science, 62 (4): 661-668, 2009). Male Crl: CDl (ICR) mice (Charles River Laboratories Japan, Inc.) were used. Twenty-one days after the administration of BLM, 1×10⁶ cells/weight (kg) of Muse cells, or vehicle, were administered via their tail veins.

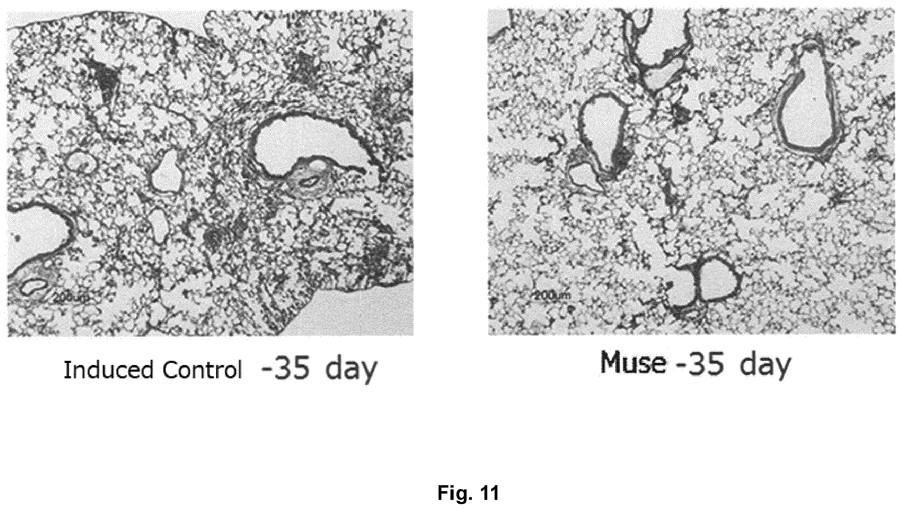

After extracting lungs were fixed with formalin, Masson's trichrome-staining samples were prepared. Using the Masson's trichrome-staining samples, scoring of fibrosis was carried out for each leaf of the lungs. Pulmonary fibrosis score was classified according to the evaluation criteria of pulmonary fibrosis described below. Then, images close to the average of the pulmonary fibrosis score of each group was taken (FIG. 11). Fibrosis scores were evaluated using lung tissues 21 days and 35 days after administration of BLM.

### Evaluation Criteria of Pulmonary Fibrosis (values represent scores)

0: normal
1: Mild fibrous thickening of alveolar or bronchial wall is observed
2: Moderate fibrous thickening of alveolar or bronchial wall is observed, but no obvious lung structural change is involved
3: Obvious lung structural changes and formation of small fibrosis lesions are observed
4: Strong lung structural changes and formation of large fibrosis lesions are observed
5: The entire lung is replaced with fibrosis

In order to examine the effect of Muse cells on increased respiration rate occurring after administration of BLM, the numbers of occurrence of increased respiration rate were counted from 21 days to 35 days after BLM administration to the Control group and the Muse cell treated group.

### Results

### 1. Pulmonary Fibrosis Score

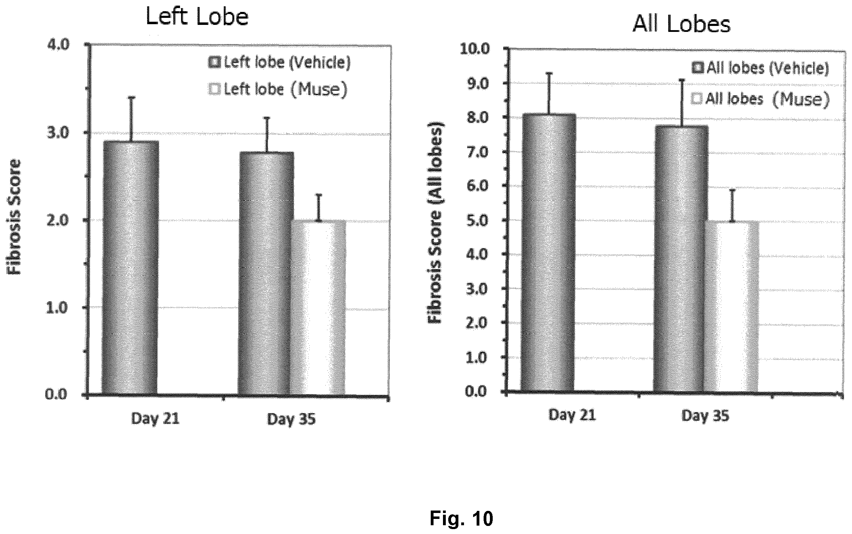

The results of pulmonary fibrosis score are shown in Fig. 10. The results showed that fibrosis had been formed 21 days after the BLM administration and the degree of the fibrosis did not change even after 35 days.

On the other hand, the Muse cell treated group had lower fibrosis score 35 days after the BLM administration as compared with the vehicle-treated group. When further comparing fibrosis scores for each pulmonary lobe, fibrosis score in left lung in Muse cell treated group tended to decrease as compared with that in vehicle treated group (p = 0.086).

### 2. Incidence of Increased Respiration Rate

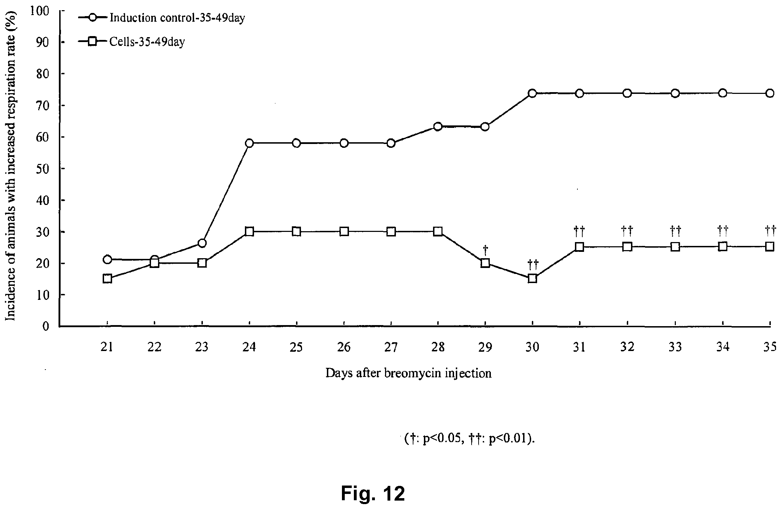

As shown in FIG. 12, the incidence of increased respiration rate was significantly decreased in the Muse cell treated group, from 29 days to 35 days after the BLM administration, as compared with the vehicle-treated group.

### Example 4

### Evaluation of Muse Cells in a Liver Fibrosis Model

Mice were administered intraperitoneally with 10 mL/kg of 10% carbon tetrachloride twice a week for 12 weeks to induce liver fibrosis. Female BALB/c mice (Charles River Laboratories Japan, Inc.) were used.

At day 57 after the initial administration of carbon tetrachloride, 1×10⁶ cells/weight (kg) (low dose group) and 1×10⁷ cells/weight (kg) (high dose group) of Muse cells were administered via their tail veins.

At day 84 after the initial administration of carbon tetrachloride, livers were extracted and subjected to HE staining and Masson's trichrome staining. Using a light microscope, the degree of fibrosis was observed.

Observed changes were graded (0, none; 1, minimal; 2, mild; 3, moderate; 4, severe) for evaluation.

Bloods were collected at day 84 after the initial administration of carbon tetrachloride, and ALT (GPT), AST (GOT), ALB (albumin), CHE (cholinesterase) and TBIL (total bilirubin) in plasma were measured using DRI-CHEM (FUJIFILM Medical Co., Ltd.).

### Results

### 1. Fibrosis Level

The degrees of fibrosis are shown in Table 1. The degrees of fibrosis in the vehicle control group were at level 2 in 10 out of 10 cases.

The degrees of fibrosis in the low-dose Muse cell group were at level 1 in 9 cases and at level 2 in 1 case out of 10 cases.

The degrees of fibrosis in the high-dose Muse cell group were at level 1 in 7 cases and at level 2 in 3 cases out of 10 cases.

Both of the groups administered with Muse cells showed significantly improved fibrosis as compared with the vehicle control group.

**[Table 1]**

| Group | | | Vehicle control | | | | | | Low dose | | | | | | | High dose | | | | | | |
|---|---|---|---|---|---|---|---|---|---|---|---|---|---|---|---|---|---|---|---|---|---|---|
| Number of animals | | | 10 | | | | | | 10 | | | | | | | 10 | | | | | | |
| Organs and findings | | | 0 | 1 | 2 | 3 | 4 | Total | 0 | 1 | 2 | 3 | 4 | Total | | 0 | 1 | 2 | 3 | 4 | Total | |
| Digestive system | | | | | | | | | | | | | | | | | | | | | | |
| | Liver | | | | | | | | | | | | | | | | | | | | | |
| | | Fibrosis | 0 | 0 | 10 | 0 | 0 | 10 | 0 | 9 | 1 | 0 | 0 | 10 | ^{∗∗} | 0 | 7 | 3 | 0 | 0 | 10 | ^{∗∗} |

| | | | | | | | | | | | | | | | | | | | | | | |
|---|---|---|---|---|---|---|---|---|---|---|---|---|---|---|---|---|---|---|---|---|---|---|
| ^{∗∗} P<0. 01 | | | | | | | | | | | | | | | | | | | | | | |

### 2. Measurement of ALT (GPT), AST (GOT), ALB, CHE and TBIL in Plasma

The vehicle control group showed changes in ALT, AST/ALT ratio, and TBIL indicating hepatitis, while both of the Muse cell treated groups (low-dose and high-dose) showed significant improvement effects on ALT, AST/ALT ratio and TBIL. Slight improvement trends were observed in ALB and CHE compared to the vehicle control group.

### Industrial Availability

The cell product of the present invention can regenerate and repair tissues in injured sites, as well as restore their functions when it is administered to fibrosis patients, and thus can be applied to prevention and treatment of fibrosis.

## Claims

1. A cell product for use in prevention and/or treatment of organ fibrosis, comprising a SSEA-3-positive pluripotent stem cell derived from mesenchymal tissue in a living body or a cultured mesenchymal cell, wherein said pluripotent stem cell shows negative expression for CD117, CD146, NG2, CD34, vWF and CD271, and wherein said organ fibrosis is a fibrosis occurring in at least one organ selected from the group consisting of a digestive organ, a respiratory organ, or on skin.

2. The cell product for use of claim 1, wherein said organ fibrosis is a fibrosis occurring in digestive organ.

3. The cell product for use of claim 2, wherein said organ fibrosis is liver fibrosis.

4. The cell product for use of claim 3, wherein said pluripotent stem cell is capable of differentiating into a cell expressing a hepatoblast marker or a hepatocyte marker.

5. The cell product for use of claim 3 or 4, which improves serum total bilirubin and/or serum albumin levels when administered to a subject, as compared with that of non-administration group.

6. The cell product for use of claim 1, wherein said organ fibrosis is a fibrosis occurring on skin.

7. The cell product for use of claim 1, wherein said organ fibrosis is a fibrosis occurring in lung.

8. The cell product for use of any one of claims 1 to 7, wherein said pluripotent stem cell has all of the following characteristics:
(i) having low or no telomerase activity;
(ii) capable of differentiating into any of tridermic cells;
(iii) showing no neoplastic proliferation; and
(iv) having self-renewal capacities.

9. A cell product for use in inhibition of tissue fibrosis and/or lysis of fibrotic tissue, comprising a SSEA-3-positive pluripotent stem cell derived from mesenchymal tissue in a living body or a cultured mesenchymal cell, wherein said pluripotent stem cell shows negative expression for CD117, CD146, NG2, CD34, vWF and CD271, and wherein said tissue is at least one tissue selected from the group consisting of a digestive tissue, a respiratory tissue and skin tissue.

## Patentansprüche

1. Zellprodukt zur Verwendung in der Vorbeugung und/oder Behandlung von Organfibrose, umfassend eine SSEA-3-positive pluripotente Stammzelle, die aus mesenchymalem Gewebe in einem lebenden Körper oder einer kultivierten mesenchymalen Zelle abgeleitet ist, wobei die pluripotente Stammzelle negative Expression für CD117, CD146, NG2, CD34, vWF und CD271 zeigt, und wobei es sich bei der Organfibrose um eine Fibrose handelt, die in mindestens einem Organ auftritt, ausgewählt aus der Gruppe bestehend aus einem Verdauungsorgan, einem Atmungsorgan, oder in der Haut.

2. Zellprodukt zur Verwendung nach Anspruch 1, wobei es sich bei der Organfibrose um eine Fibrose handelt, die in einem Verdauungsorgan auftritt.

3. Zellprodukt zur Verwendung nach Anspruch 2, wobei es sich bei der Organfibrose um Leberfibrose handelt.

4. Zellprodukt zur Verwendung nach Anspruch 3, wobei die pluripotente Stammzelle in der Lage ist, sich in eine Zelle zu differenzieren, die einen Hepatoblastenmarker oder einen Hepatozytenmarker exprimiert.

5. Zellprodukt zur Verwendung nach Anspruch 3 oder 4, das, wenn es einem Subjekt verabreicht wird, das Gesamtbilirubin im Serum und/oder Serumalbuminspiegel verglichen mit denen einer Nichtverabreichungsgruppe verbessert.

6. Zellprodukt zur Verwendung nach Anspruch 1, wobei es sich bei der Organfibrose um eine Fibrose handelt, die in der Haut auftritt.

7. Zellprodukt zur Verwendung nach Anspruch 1, wobei es sich bei der Organfibrose um eine Fibrose handelt, die in der Lunge auftritt.

8. Zellprodukt zur Verwendung nach einem der Ansprüche 1 bis 7, wobei die pluripotente Stammzelle alle der folgenden Eigenschaften aufweist:
(i) geringe oder keine Telomeraseaktivität aufweisend;
(ii) in der Lage, sich in jede tridermale Zelle zu differenzieren;
(iii) keine neoplastische Proliferation zeigend; und
(iv) Selbsterneuerungsfähigkeiten aufweisend.

9. Zellprodukt zur Verwendung in der Hemmung von Gewebefibrose und/oder Lyse von fibrotischem Gewebe, umfassend eine SSEA-3-positive pluripotente Stammzelle, die aus mesenchymalem Gewebe in einem lebenden Körper oder einer kultivierten mesenchymalen Zelle abgeleitet ist, wobei die pluripotente Stammzelle negative Expression für CD117, CD146, NG2, CD34, vWF und CD271 zeigt, und wobei das Gewebe mindestens ein Gewebe ist, ausgewählt aus der Gruppe bestehend aus einem Verdauungsgewebe, einem Atmungsgewebe und Hautgewebe.

## Revendications

1. Produit cellulaire à usage préventif et/ou pour le traitement de la fibrose d'organe, comprenant une cellule souche pluripotente SSEA-3 positive dérivée de tissu mésenchymateux dans un corps vivant ou une cellule mésenchymateuse cultivée, dans lequel ladite cellule souche pluripotente présente une expression négative pour CD117, CD146, NG2, CD34, vWF et CD271, et dans lequel ladite fibrose d'organe est une fibrose se produisant dans au moins un organe choisi dans le groupe constitué d'un organe digestif, d'un organe respiratoire ou sur la peau.

2. Produit cellulaire pour l'utilisation selon la revendication 1, dans lequel ladite fibrose d'organe est une fibrose se produisant dans un organe digestif.

3. Produit cellulaire pour l'utilisation selon la revendication 2, dans lequel ladite fibrose d'organe est une fibrose hépatique.

4. Produit cellulaire pour l'utilisation selon la revendication 3, dans lequel ladite cellule souche pluripotente est capable de se différencier en une cellule exprimant un marqueur d'hépatoblaste ou un marqueur d'hépatocyte.

5. Produit cellulaire pour l'utilisation selon la revendication 3 ou 4, qui améliore des niveaux de bilirubine totale sérique et/ou d'albumine sérique lorsqu'il est administré à un sujet, par rapport à ceux du groupe sans administration.

6. Produit cellulaire pour l'utilisation selon la revendication 1, dans lequel ladite fibrose d'organe est une fibrose se produisant sur la peau.

7. Produit cellulaire pour l'utilisation selon la revendication 1, dans lequel ladite fibrose d'organe est une fibrose se produisant dans le poumon.

8. Produit cellulaire pour l'utilisation selon l'une quelconque des revendications 1 à 7, dans lequel ladite cellule souche pluripotente présente toutes les caractéristiques suivantes :
(i) présentant une activité télomérase faible ou nulle ;
(ii) capable de se différencier en n'importe laquelle des cellules tridermiques ;
(iii) ne montrant aucune prolifération néoplasique ; et
(iv) présentant des capacités d'auto-renouvellement.

9. Produit cellulaire pour l'utilisation dans l'inhibition de la fibrose tissulaire et/ou lyse de tissu fibrotique, comprenant une cellule souche pluripotente SSEA-3 positive dérivée d'un tissu mésenchymateux dans un corps vivant ou une cellule mésenchymateuse cultivée, ladite cellule souche pluripotente présentant une expression négative pour CD117, CD146, NG2, CD34, vWF et CD271, et dans lequel ledit tissu est au moins un tissu choisi dans le groupe constitué d'un tissu digestif, d'un tissu respiratoire et d'un tissu cutané.
